# FASCICULE DE BREVET EUROPEEN

(11) **EP 2 575 693 B1**
(45) Date de publication et mention de la délivrance du brevet: **11.04.2018**
(21) Numéro de dépôt: 11728322.6
(22) Date de dépôt: 20.05.2011
(51) Int. Cl.: A61F 2/78, A61F 2/80, A61F 7/00, A61F 2/70, A61F 2/76

(54) **DISPOSITIF DE CHAUFFAGE ET/OU DE REGULATION DE TEMPERATURE POUR EMBOITURES DE PROTHESES**
VORRICHTUNG ZUR ERWÄRMUNG UND/ODER TEMPERATURREGELUNG EINES PROTHESENSCHAFTES
DEVICE FOR THE HEATING AND/OR TEMPERATURE CONTROL OF PROSTHESIS SOCKETS

(30) Priorité: 07.06.2010 FR 1054439
(43) Date de publication de la demande: 10.04.2013
(73) Titulaire: Allemand, André Marcel Emile, 66550 Corneilla La Riviere (FR)
(72) Inventeur: Allemand, André Marcel Emile, 66550 Corneilla La Riviere (FR)
(74) Mandataire: Pontet Allano & Associes
(86) Numéro de dépôt international: PCT/FR2011/051155
(87) Numéro de publication internationale: WO 2011/154633

(56) Documents cités:
- WO-A2-2010/129334
- GB-A- 2 407 961
- US-A- 5 139 523
- US-A- 5 913 849
- US-A1- 2004 260 402

## Description

### Domaine technique

La présente invention concerne un dispositif de chauffage et ou de régulation de la température de l'emboîture d'un appareillage orthopédique destiné à équiper un moignon d'amputation.

Le domaine de l'invention est celui de l'appareillage orthopédique et des prothèses à destination des personnes amputées.

### Etat de la technique antérieure

Les personnes amputées d'un membre souffrent fréquemment de douleurs récurrentes, appelées également douleur du membre fantôme, qui peuvent être très gênantes.

Comme décrit dans l'article de H. Flor, N. Birbaumer and R.A. Sherman, « Phantom Limb Pain », Pain: Clinical Updates, Volume VIII, No. 3, June 2000, ces douleurs proviennent du fait que les terminaisons nerveuses du moignon, c'est-à-dire de la zone dans laquelle le membre a été sectionné, demeurent sensibles aux stimuli tels que par exemple les irritations et les frottements liés à l'activité du moignon dans la prothèse, et au froid.

Il est également montré dans cet article que ces états douloureux sont souvent liés à une réduction du débit sanguin superficiel sur la partie distale des moignons et aux conséquences que cela entraîne sur la régulation thermique de cette partie.

En effet, la réponse physiologique du corps aux variations de température extérieures consiste normalement en une régulation de la température cutanée, de telle sorte à la maintenir dans une zone de neutralité thermique sensiblement comprise entre 30°C et 33°C, par vasodilatation ou vasoconstriction des vaisseaux sanguins superficiels selon qu'il s'agit d'une exposition au froid ou au chaud.

Dans le cas de la zone amputée, lorsque le mécanisme de vasodilatation est défaillant la température n'est plus régulée correctement. Il a été constaté expérimentalement qu'en cas d'exposition au froid, cette température superficielle pouvait être inférieure de l'ordre de 4 à 5°C à la température de la zone équivalente (c'est-à-dire à la même position) du membre sain.

Les terminaisons nerveuses lésées sont donc soumises au froid, ce qui augmente le rythme des décharges et déclenche ou accentue fortement les sensations de douleur.

Pour de nombreux amputés, les douleurs peuvent être soulagées en réchauffant la zone distale du moignon d'amputation, par exemple dans de l'eau chaude. Mais cette méthode nécessite de retirer tout appareillage et n'est donc utilisable qu'au repos.

On connait les patchs chauffants, comme par exemple celui décrit dans le document US 5,913,849, qui sont destinés à être appliqués à même la peau pour chauffer localement une zone du corps. Cette solution est applicable temporairement pour soulager des douleurs, mais uniquement dans la mesure où le moignon d'amputation n'est pas inséré dans un appareillage d'orthopédie tel qu'une prothèse, auquel cas les frottements et les appuis la rendraient insupportable.

Or le problème d'éviter la stimulation des terminaisons nerveuses dans le moignon est particulièrement important dans le cadre de l'appareillage du membre amputé avec une prothèse ou un quelconque appareillage orthopédique. En effet, la zone distale du moignon amputé et les tissus cicatriciels sont directement en contact avec des éléments de l'emboîture. Ces éléments sont constitués en général de pièces en silicone souples qui évitent les stimulations mécaniques excessives du moignon, et de pièces en matériaux composites et/ou métalliques pour les parties rigides. Ces éléments de la prothèse sont soumis à la température extérieure et la communiquent à la surface du moignon, avec lequel ils sont en contact étroit, par conduction thermique.

On connait le document US 5 139 523 A qui décrit un enroulement résistif apte a être disposé dans l'emboîture d'un appareillage orthopédique à des fins de chauffage. On connait également le document WO 2010/129334 A2 (relevant de l'article 54(3) CBE) qui décrit un liner muni d'éléments chauffants apte à être disposé dans un appareillage orthopédique de réception d'un moignon d'amputation de telle sorte à chauffer des parties de l'emboiture dudit appareillage en contact avec l'extrémité du moignon d'amputation.

Le but de la présente invention est de proposer un dispositif pouvant être adapté à des appareillages orthopédiques existants, qui permette d'en améliorer sensiblement le confort en évitant une stimulation des terminaisons nerveuses du moignon sous l'effet du froid.

### Exposé de l'invention

Cet objectif est atteint avec un dispositif selon la revendication 1.

Lorsque le moignon est inséré dans son appareillage orthopédique, les parties sensibles comprenant les terminaisons nerveuses et les tissus cicatriciels sont maintenus à une température permettant de minimiser les stimulations des nerfs, et ceci indépendamment de la température extérieure.

En outre, le fait de chauffer et/ou de réguler en température les parties de l'emboîture déjà prévues pour être en contact avec l'extrémité distale du moignon présente l'avantage d'éviter d'amener des éléments supplémentaires en contact avec le moignon, qui seraient susceptibles de poser des problèmes de tolérance additionnels (appuis, matériau, frottements, ...).

L'élément chauffant peut être un élément inséré dans un appareillage orthopédique. Ce peut être également une pièce de l'emboîture qui comprend des moyens de chauffage tels que des pistes chauffantes, des corps de chauffe ou des résistances électriques, auquel cas l'élément chauffant est inclus dans une pièce de l'appareillage orthopédique.

L'élément chauffant a la forme d'un film souple, lequel film comprenant un support en matériau souple sensiblement isolant à l'électricité et, sur au moins une surface dudit support, des pistes en matériau sensiblement conducteur à l'électricité aptes à s'échauffer sous l'effet du passage d'un courant électrique. Ces pistes peuvent par exemple comprendre un alliage métallique avec une résistivité relativement élevée tel que de l'Inconel® ou du cupronickel (un alliage comprenant du cuivre et du nickel), ou même du cuivre.

L'élément chauffant peut être réalisé par exemple sous la forme d'un circuit imprimé souple sur lequel un réseau de pistes est gravé ou déposé selon un procédé classique en électronique tel que le masquage, la gravure chimique ou la sérigraphie. Ce réseau de piste peut comprendre une seule ou une pluralité de pistes, réparties de telle sorte à couvrir au moins une partie de la surface de l'élément chauffant.

Sous l'action du passage d'un courant électrique, le réseau de pistes qui peuvent être réparties selon des configurations électriques en série et/ou en parallèle s'échauffe sous l'effet des pertes ohmiques, ce qui produit la chaleur recherchée. La répartition des pistes sur la surface, ainsi que leur largeur locale, peuvent être ajustées pour générer des profils de température particuliers à la surface de l'élément chauffant. Pour la plupart des applications, toutefois une répartition sensiblement homogène des pistes sur au moins une partie de la surface de l'élément chauffant est préférable.

Le support de l'élément chauffant peut comprendre l'un des matériaux suivants : polyimide, Kapton®, téflon, caoutchouc de silicone, ou tout matériau souple adapté.

Le polyimide ou le Kapton® présentent notamment l'avantage de pouvoir être à la fois très minces tout en présentant une bonne résistance aux contraintes mécaniques. Ils permettent de réaliser des éléments chauffants qui peuvent être insérés entre des pièces de l'emboîture d'un appareillage orthopédique déjà existant, ou dans lequel cette fonction n'a pas été nécessairement prévue lors de la conception, sans en perturber le fonctionnement.

L'élément chauffant peut comprendre en outre une couche métallique réfléchissante, apte à réfléchir un rayonnement thermique, telle qu'une feuille aluminisée. Cette couche peut être de préférence disposée sur la face de l'élément chauffant opposée au moignon d'amputation. Elle permet d'améliorer les transferts de chaleurs en direction du moignon tout en limitant les déperditions dans le corps de l'appareillage orthopédique.

D'autres couches de matériaux sensiblement isolants thermiquement, sous toute forme, peuvent bien entendu être ajoutées pour limiter au mieux les déperditions thermiques.

L'élément chauffant peut comprendre en outre selon l'une de ses faces (au moins) une couche adhésive, de telle sorte à pouvoir être aisément fixé sur un élément de l'emboîture. Cette couche adhésive peut par exemple être disposée sur la face opposée à la face supportant la couche métallique réfléchissante. Selon la présente invention, l'élément chauffant comprend des découpes le rendant apte à être collé sur une surface incurvée, tout en minimisant les épaisseurs et les plis.

En particulier, il est de forme sensiblement circulaire ou elliptique, et comprend des découpes selon des orientations radiales. Il peut être ainsi sensiblement en forme de fleur avec une partie centrale et une pluralité de « pétales », ce qui le rend particulièrement adapté à un collage sur des éléments d'emboîture qui épousent l'extrémité distale d'un manchon d'amputation et qui sont en général d'une forme sensiblement hémisphérique.

Le dispositif selon l'invention peut comprendre en outre un capteur de température disposé de telle sorte à mesurer la température au voisinage de l'élément chauffant, et des moyens de régulation de température aptes à maintenir ledit élément chauffant à une température de consigne.

Le capteur de température peut être placé de telle sorte à mesurer une température représentative de la température des parois de l'emboîture en contact avec le moignon.

Le capteur de température peut comprendre l'un des éléments suivants : résistance de platine, PT100, thermistance, thermocouple, ou tout autre type d'élément sensible. Cet élément peut être disposé sur l'élément chauffant. En particulier, le capteur de température peut comprendre un élément de très faible épaisseur, tel qu'un composant à montage de surface (CMS), soudé sur l'élément chauffant.

Ce mode de réalisation permet d'obtenir un élément chauffant qui se présente sous la forme d'un circuit imprimé souple sans aucun composant additionnel en surépaisseur, ce qui lui confère une résistance mécanique et des possibilités d'intégration optimales. En outre le capteur de température peut être placé vers le centre de l'élément vis-à-vis du moignon, ce qui offre des conditions de mesure idéales.

Le dispositif selon l'invention peut comprendre en outre au moins un autre capteur de température mesurant par exemple la température ambiante, ou la température de la peau dans une zone autre que celle du moignon.

Les moyens de régulation peuvent comprendre un thermostat simple, qui déclenche ou interrompt l'alimentation électrique de l'élément chauffant lorsque la température mesurée franchit un seuil. Ils peuvent également comprendre un dispositif à microcontrôleur qui optimise l'alimentation de l'élément chauffant en fonction de la température mesurée et éventuellement d'autres paramètres tels que la température extérieure, la température de la peau mesurée dans une zone autre que le moignon, la durée de vie ou l'état des batteries d'alimentation, ...

Le dispositif selon l'invention peut être conçu de telle sorte à pouvoir maintenir des températures de consigne de l'ordre de 30 à 40°C. En particulier, la température de consigne peut être sensiblement comprise entre 30°C et 33°C, ce qui correspond à la température « normale » à la surface de la peau lorsque les mécanismes de régulation physiologiques fonctionnent correctement.

Le dispositif selon l'invention peut comprendre en outre un boîtier apte à être porté par l'utilisateur et relié à l'élément chauffant par un câble de liaison, lequel boîtier comprenant des batteries d'alimentation, et des moyens de réglage de la température de consigne par l'utilisateur.

Les batteries d'alimentation peuvent être par exemple de type lithium-ion ou lithium-polymère.

Le boîtier peut comprendre des moyens d'accrochage comme un clip pour le porter à la ceinture, ou des moyens pour le porter en bandoulière ou pour l'accrocher à l'appareillage orthopédique.

Le câble de liaison peut comprendre des moyens de connectique permettant de déconnecter aisément le boîtier, par exemple lorsque l'utilisateur change de prothèse (auquel cas l'élément chauffant reste dans le prothèse) ou en cas de remplacement de l'élément chauffant.

Suivant un mode de réalisation, l'élément chauffant peut être inséré entre l'emboîture souple et l'emboîture rigide d'une prothèse de membre, et collé à ladite emboîture souple.

Suivant un autre aspect de l'invention, il est proposé un appareillage orthopédique comprenant une emboîture destinée à recevoir l'extrémité distale d'un moignon d'amputation, selon la revendication 12.

Cet appareillage orthopédique peut être de l'un des types suivants : Prothèse de membre inférieur pour l'appareillage d'un moignon d'amputation tibial, prothèse de membre inférieur pour l'appareillage d'un moignon d'amputation fémoral, prothèse de membre supérieur.

Suivant encore un autre aspect de l'invention, il est proposé un procédé d'amélioration du confort du port d'un appareillage orthopédique comprenant une emboîture destinée à recevoir l'extrémité distale d'un moignon d'amputation, caractérisé en ce qu'il comprend une opération de chauffage et/ou de maintien à une température prédéterminée de parties de ladite emboîture en contact avec ladite extrémité distale du moignon d'amputation, au moyen d'un élément chauffant disposé dans ledit appareillage orthopédique.

La température prédéterminée peut être sensiblement égale à la température de la surface de la peau dans une zone saine de localisation anatomique sensiblement équivalente à celle du moignon d'amputation, dans laquelle les mécanismes de régulation physiologiques de la température fonctionnent correctement.

### Description des figures et modes de réalisation

D'autres avantages et particularités de l'invention apparaîtront à la lecture de la description détaillée de mises en oeuvre et de modes de réalisation nullement limitatifs, et des dessins annexés suivants :
- la figure 1 illustre un mode de réalisation de dispositif selon l'invention,
- la figure 2 montre une vue de dessus de l'élément chauffant,
- la figure 3 illustre une vue en coupe de l'élément chauffant.

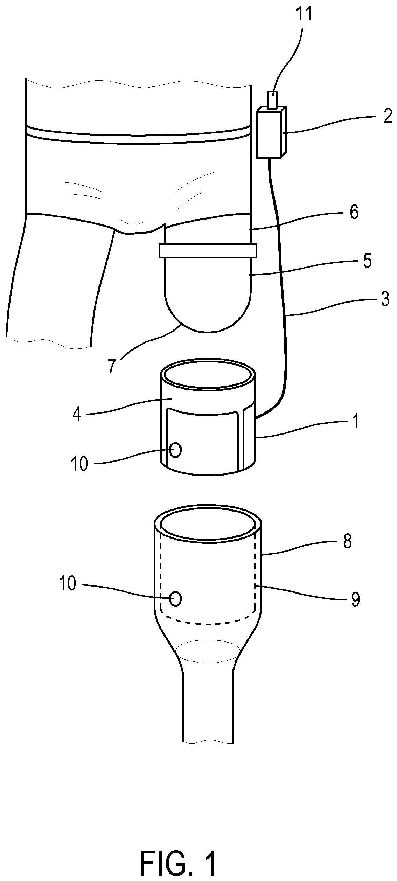

En référence à la figure 1, on va décrire un mode de réalisation d'un dispositif selon l'invention pour l'équipement d'une prothèse fémorale.

Ces prothèses sont destinées à appareiller des moignons d'amputation 6 sectionnés entre le genou et la hanche. Le moignon d'amputation 6 comprend une extrémité distale 7, qui correspond à l'endroit de l'amputation. C'est dans cette extrémité distale 7 que se situent la zone de tissus cicatriciels et les terminaisons nerveuses lésées par suite de l'amputation.

L'extrémité distale 7 du moignon d'amputation 6 est en général recouverte d'un manchon 5 en silicone qui la recouvre et la protège.

Suivant un mode de réalisation commun à la plupart des prothèses de membres, la prothèse fémorale comprend une emboîture souple 4, qui est fixée par emboîtement ou tout autre moyen dans l'emboîture rigide 9 de la prothèse 8. Le manchon 5 s'emboîte de manière sensiblement étanche dans l'emboîture souple 4. Une valve 10 permet à l'air de s'échapper de l'emboîture souple 4 lors de la mise en place du manchon 5. Cette valve 10 est ensuite refermée et la prothèse 8 est maintenue en place par la dépression. Pour enlever la prothèse, il suffit de laisser entrer de l'air par la valve 10.

L'élément chauffant 1 du dispositif selon l'invention est réalisé sous la forme d'un film souple, qui est collé autour de la base de l'emboîture souple 4. Il est disposé de manière à chauffer de manière sensiblement homogène la partie constituant la base de l'emboîture souple 4 qui est directement en regard de l'extrémité distale 7 du moignon d'amputation 6. De cette manière, il est possible de chauffer ou maintenir à une température déterminée de manière très homogène l'ensemble de zone distale 7 comprenant la partie cicatricielle sensible du moignon d'amputation 6.

La localisation de cet élément chauffant 1 dans l'emboîture est également choisie de telle manière à ne pas perturber le fonctionnement des parties de l'emboîture telles que le manchon 5 et l'emboîture souple 4, qui sont en contact avec le moignon d'amputation 6 ou à proximité immédiate de ce dernier, et qui déterminent le confort et la façon dont la prothèse 8 sera tolérée.

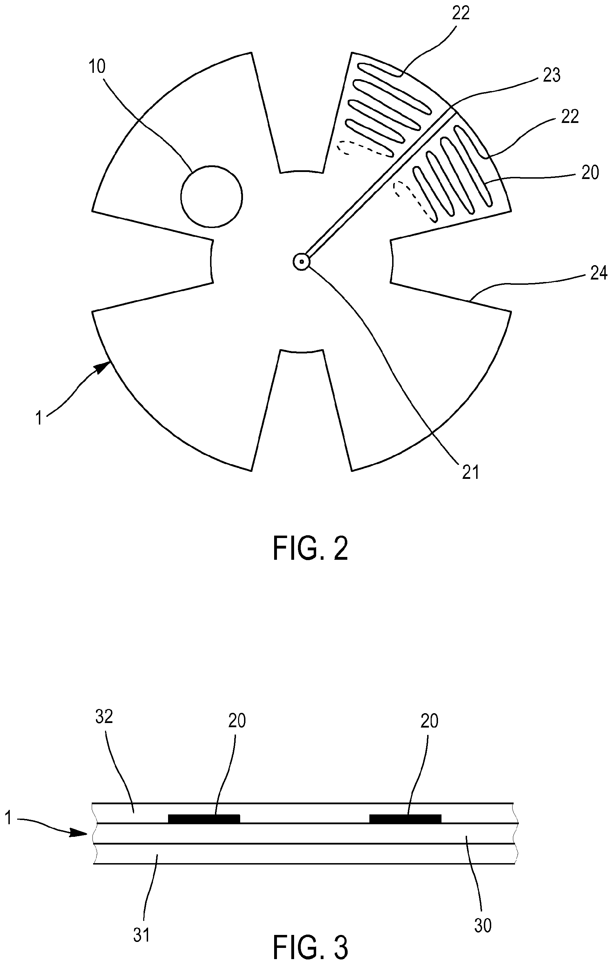

En référence aux figures 2 et 3, l'élément chauffant 1 est réalisé sur la base d'une technologie de circuit imprimé souple. Il comprend un support 30 en polyimide ou Kapton®, sur lequel sont gravées des pistes 20 constituées d'alliage métallique tel que de l'Inconel® ou du cupronickel. Le matériau du support 30 est choisi en fonction de ses qualités de souplesse et de résistance mécanique. Les pistes sont obtenues selon un procédé classique en électronique, par exemple de gravure.

La surface utile de l'élément chauffant 1 est recouverte par un réseau de pistes 20 réparties de manière sensiblement uniforme. Seule une partie de ce réseau est représentée à la figure 1 pour des raisons de lisibilité. Les pistes sont reliées électriquement à des moyens d'alimentation électrique par des points de connexion 22.

Le réseau de piste est calculé de telle sorte à présenter une résistance R de l'ordre de 30 à 50 Ohm entre les points de connexion 22. Ainsi, lorsqu'il est alimenté par une tension **U** = 11.1 volt, l'élément chauffant dissipe sous forme de chaleur une puissance **P = U**^2 / **R** de l'ordre de 3.5 watt.

L'élément chauffant 1 comprend en outre un capteur de température 21 disposé vers son centre, à une position où la température mesurée est particulièrement représentative de celle à laquelle est soumise l'extrémité distale 7 du moignon d'amputation 6. Ce capteur de température 21 est relié à des moyens de contrôle par des points de connexion 23. Le capteur de température 21 est de type à résistance de platine (PT100), et se présente sous la forme d'un composant à montage de surface (CMS) de très faibles dimensions et surtout de très faible hauteur, inférieure à 0.5 mm.

Dans la mesure où l'élément chauffant 1 est destiné à être inséré entre des pièces de l'emboîture de la prothèse sans en gêner le fonctionnement tout en étant soumis à des contraintes mécaniques potentiellement fortes, il est important qu'il soit réalisé sous la forme d'un film mince et résistant, sans éléments notablement saillants.

L'élément chauffant 1 comprend des découpes 24. La forme de ces découpes 24 peut bien entendu être librement optimisée pour chaque application. Le mode de réalisation de la figure 2 est adapté à l'insertion dans la prothèse fémorale de la figure 1. Les découpes définissent des pétales qui peuvent être rabattus et collés sur une surface sensiblement hémisphérique sans plissement excessif de l'élément chauffant 1.

L'élément chauffant 1 comprend également une ouverture 10 pour le passage de la valve qui assure l'étanchéité de l'emboîtement du manchon 5 dans l'emboîture souple 4.

La figure 3 présente une vue en coupe de l'élément chauffant 1. Cette représentation est purement schématique et les épaisseurs relatives des couches dessinées ne sont pas représentatives des dimensions réelles. L'épaisseur réelle totale de l'élément chauffant peut être sensiblement inférieure à 1 mm, voire inférieure à 0.5 mm.

L'élément chauffant 1 comprend sur ses faces une couche adhésive de colle 32. Il peut ainsi être aisément mis en place par collage sur l'emboîture souple 4, et au besoin remplacé.

L'élément chauffant 1 comprend également une couche réfléchissante 31, telle qu'un film d'aluminium mince, collée sur la face orientée vers l'emboîture rigide 9. La fonction de cette couche réfléchissante 31 est d'améliorer l'efficacité thermique en réfléchissant le rayonnement thermique généré de manière sensiblement isotrope par les pistes 20 en direction du moignon 6.

L'élément chauffant 1 est relié par un câble de liaison 3 et un connecteur à un boitier de contrôle 2 qui peut être par exemple fixé à la ceinture de l'utilisateur par un clip. Ce boîtier 2 comprend les batteries d'alimentation, de type lithium-ion ou lithium polymère par exemple, ainsi que l'électronique de contrôle et de régulation de la température, basée sur un microcontrôleur. Il comprend également une possibilité de réglage 11 de la température de l'élément chauffant 1 par l'utilisateur.

La puissance de l'élément chauffant est calculée de telle sorte à permettre une élévation de la température de la partie distale 7 du moignon d'amputation de l'ordre de 4°C au moins dans l'environnement de la prothèse, de telle sorte à maintenir cette partie dans la zone de neutralité thermique, soit environ 30 à 33°C. Dans ces conditions, la capacité des batteries permet une autonomie de fonctionnement de plus de 6 heures.

Le réglage de la température permet à l'utilisateur d'augmenter la température de consigne jusqu'à 40°C au moins pendant des courtes durées, ce qui peut lui permettre d'atténuer et de soulager un accès de douleur, ou simplement de choisir une température de confort différente.

Suivant des modes de réalisation :
- Les pistes ou corps de chauffe 20 peuvent être réalisées par tout procédé, parmi lesquels la sérigraphie ;
- L'élément chauffant 1 peut comprendre un support 30 moulé selon une forme particulière, par exemple pour épouser au mieux la forme d'une surface particulière ;
- L'élément chauffant 1 peut être de toute forme, et comprendre tout type de découpes, en fonction de la prothèse à laquelle il est destiné ;
- L'élément chauffant 1 peut être intégré à un élément de la prothèse tel que l'emboîture souple 4 ou l'emboîture rigide 9, ou le corps 8, durant sa fabrication, notamment par l'incorporation d'éléments chauffants dans cet élément.

Bien sûr, l'invention n'est pas limitée aux exemples qui viennent d'être décrits et de nombreux aménagements peuvent être apportés à ces exemples sans sortir du cadre de l'invention telle que définie dans les revendications.

## Revendications

1. Dispositif de chauffage et/ou de régulation de la température de l'emboîture d'un appareillage orthopédique (8) destinée à recevoir l'extrémité distale (7) d'un moignon d'amputation (6), comprenant un élément chauffant (1) apte à être disposé dans ledit appareillage orthopédique (8) de telle sorte à chauffer et/ou à maintenir à une température prédéterminée des parties (4, 5) de ladite emboîture en contact avec ladite extrémité distale (7) du moignon d'amputation (6), ledit élément chauffant (1) a la forme d'un film souple, lequel film comprenant un support (30) en matériau souple sensiblement isolant à l'électricité et, sur au moins une surface dudit support (30), des pistes (20) en matériau sensiblement conducteur à l'électricité aptes à s'échauffer sous l'effet du passage d'un courant électrique ;
**caractérisé en ce que** :
- l'élément chauffant (1) comprend des découpes (24) selon des orientations radiales le rendant apte à être collé sur une surface incurvée, et
- l'élément chauffant (1) est de forme sensiblement circulaire ou elliptique.

2. Dispositif selon la revendication 1, **caractérisé en ce que** le support (30) comprend l'un des matériaux suivants : polyimide, Kapton®, caoutchouc de silicone.

3. Dispositif selon l'une des revendications 1 ou 2, **caractérisé en ce que** l'élément chauffant (1) comprend en outre une couche métallique réfléchissante (31), apte à réfléchir un rayonnement thermique.

4. Dispositif selon l'une des revendications 1 à 3, **caractérisé en ce que** l'élément chauffant (1) comprend en outre selon l'une de ses faces une couche adhésive (32).

5. Dispositif selon l'une des revendications 1 à 4, **caractérisé en ce qu'**il comprend en outre un capteur de température (21) disposé de telle sorte à mesurer la température au voisinage de l'élément chauffant (1), et des moyens de régulation de température aptes à maintenir ledit élément chauffant (1) à une température de consigne.

6. Dispositif selon la revendication 5, **caractérisé en ce que** le capteur de température (21) comprend l'un des éléments suivants : résistance de platine, PT100, thermistance, thermocouple, disposé sur l'élément chauffant (1).

7. Dispositif selon l'une des revendications 5 ou 6, **caractérisé en ce que** la température de consigne est sensiblement comprise entre 30°C et 33°C.

8. Dispositif selon l'une des revendications 5 à 7, **caractérisé en ce qu'**il comprend en outre un boîtier (2) apte à être porté par l'utilisateur et relié à l'élément chauffant (1) par un câble de liaison (3), lequel boîtier comprenant des batteries d'alimentation, et des moyens de réglage (11) de la température de consigne par l'utilisateur.

9. Dispositif selon l'une des revendications 1 à 8, **caractérisé en ce que** l'élément chauffant (1) est apte à être inséré entre l'emboîture souple (4) et l'emboîture rigide (9) d'une prothèse de membre (8), et apte à être collé à ladite emboîture souple (4).

10. Appareillage orthopédique comprenant une emboîture destinée à recevoir l'extrémité distale (7) d'un moignon d'amputation (6) et un dispositif de chauffage et/ou de régulation de la température de ladite emboîture selon l'une quelconque des revendications 1 à 9.

11. Appareillage orthopédique selon la revendication 10, **caractérisé en ce qu'**il est de l'un des types suivants : prothèse de membre inférieur pour l'appareillage d'un moignon d'amputation tibial, prothèse de membre inférieur pour l'appareillage d'un moignon d'amputation fémoral, prothèse de membre supérieur.

## Patentansprüche

1. Vorrichtung zur Heizung und/oder zur Regelung der Temperatur des Schafts einer orthopädischen Vorrichtung (8) zur Aufnahme des distalen Endes (7) eines Amputationsstumpfs (6), ein in der orthopädischen Vorrichtung (8) derart lagerbares Heizelement (1) umfassend, dass Teile (4, 5) des in Berührung mit dem distalen Ende (7) des Amputationsstumpfs (6) angeordneten Schafts geheizt und/oder bei einer vorbestimmten Temperatur gehalten werden, wobei das Heizelement (1) als Weichfolie ausgebildet ist, wobei die Folie einen Träger (30) aus einem weichen, im Wesentlichen elektrisch isolierenden Werkstoff und auf mindestens einer Oberfläche des Trägers (30) Bahnen (20) aus einem im Wesentlichen elektrisch leitenden Werkstoff umfasst, welche unter der Wirkung eines fließenden elektrischen Stroms erhitzbar sind;
**dadurch gekennzeichnet, dass**:
- das Heizelement (1) radial ausgerichtete Aussparungen (24) umfasst, wodurch es auf eine gekrümmte Oberfläche klebbar ist, und
- das Heizelement (1) im Wesentlichen kreisförmig oder elliptisch ausgebildet ist.

2. Vorrichtung nach Anspruch 1, **dadurch gekennzeichnet, dass** der Träger (30) einen der folgenden Werkstoffe umfasst: Polyimid, Kapton®, Silikonkautschuk.

3. Vorrichtung nach einem der Ansprüche 1 oder 2, **dadurch gekennzeichnet, dass** das Heizelement (1) außerdem eine spiegelnde Metallschicht (31) umfasst, welche zur Reflektion einer Wärmestrahlung geeignet ist.

4. Vorrichtung nach einem der Ansprüche 1 bis 3, **dadurch gekennzeichnet, dass** das Heizelement (1) außerdem je nach einer seiner Oberflächen eine Klebschicht (32) umfasst.

5. Vorrichtung nach einem der Ansprüche 1 bis 4, **dadurch gekennzeichnet, dass** sie außerdem einen derart gelagerten Temperatursensor (21) umfasst, dass er die Temperatur in Nachbarschaft des Heizelements (1) misst, sowie Temperaturregelungsmittel, die dazu geeignet sind, das Heizelement (1) bei einer Soll-Temperatur zu halten.

6. Vorrichtung nach Anspruch 5, **dadurch gekennzeichnet, dass** der Temperatursensor (21) eins der folgenden Bauelemente umfasst: Platinwiderstand, PT100, Thermistor, Thermoelement, auf dem Heizelement (1) gelagert.

7. Vorrichtung nach einem der Ansprüche 5 oder 6, **dadurch gekennzeichnet, dass** die Soll-Temperatur im Wesentlichen zwischen 30 °C und 33 °C liegt.

8. Vorrichtung nach einem der Ansprüche 5 bis 7, **dadurch gekennzeichnet, dass** sie außerdem ein durch den Benutzer tragbares, mit dem Heizelement (1) über ein Verbindungskabel (3) verbundenes Gehäuse (2) umfasst, wobei das Gehäuse Versorgungsbatterien umfasst, sowie Regelungsmittel (11) zur Regelung der Soll-Temperatur durch den Benutzer.

9. Vorrichtung nach einem der Ansprüche 1 bis 8, **dadurch gekennzeichnet, dass** das Heizelement (1) zwischen dem weichen Schaft (4) und dem steifen Schaft (9) einer Prothese für ein Körperglied (8) einsetzbar sowie an dem weichen Schaft (4) klebbar ist.

10. Orthopädische Vorrichtung, einen Schaft zur Aufnahme des distalen Endes (7) eines Amputationsstumpfs (6) und eine Vorrichtung zur Heizung und/oder zur Regelung der Temperatur des Schafts nach einem der Ansprüche 1 bis 9 umfassend.

11. Orthopädische Vorrichtung nach Anspruch 10, **dadurch gekennzeichnet, dass** sie von einem der folgenden Typen ist: Beinprothese für die Vorrichtung für einen Tibial-Amputationsstumpf, Beinprothese für die Vorrichtung für einen Femoral-Amputationsstumpf, Armprothese.

## Claims

1. A device for heating and/or controlling the temperature of the socket of an orthopaedic appliance (8) for receiving the distal end (7) of an amputation stump (6), comprising a heating element (1) arranged in said orthopaedic appliance (8) such that the parts (4, 5) of said socket in contact with said distal end (7) of the amputation stump (6) are heated and/or maintained at a predetermined temperature, said heating element (1) is in the form of a flexible film, which film comprises a support (30) of flexible substantially electrically insulating material and, on at least one surface of said support (30), tracks (20) of substantially electrically conducting material capable of being heated under the effect of an electric current passing there through;
**characterised in that** :
- the heating element (1) comprises cutouts (24) at radial orientations making it capable of being glued on a curved surface, and
- the heating element (1) is of substantially circular or elliptic shape.

2. The device according to claim 1, **characterised in that** the support (30) comprises one of the following materials: polyimide, Kapton®, silicone rubber.

3. The device according to one of claims 1 or 2, **characterised in that** the heating element (1) further comprises a reflective metal layer (31), capable of reflecting a heat radiation.

4. The device according to one of claims 1 to 3, **characterised in that** the heating element (1) further comprises an adhesive layer (32) on one of its faces.

5. The device according to one of claims 1 to 4, **characterised in that** it further comprises a temperature sensor (21) arranged so as to measure the temperature in the vicinity of the heating element (1), and temperature controlling means capable of maintaining said heating element (1) to a temperature setpoint.

6. The device according to claim 5, **characterised in that** the temperature sensor (21) comprises one of the following elements: platinum resistor, PT100, thermistor, thermocouple, arranged on the heating element (1).

7. The device according to one of claims 5 or 6, **characterised in that** the temperature setpoint is substantially between 30°C and 33°C.

8. The device according to one of claims 5 to 7, **characterised in that** it further comprises a case (2) capable of being worn by the user and connected to the heating element (1) by a connecting cable (3), which case comprises power supply batteries, and means (11) for adjusting the temperature setpoint by the user.

9. The device according to one of claims 1 to 8, **characterised in that** the heating element (1) is capable of being inserted between the flexible socket (4) and the rigid socket (9) of a limb prosthesis (8), and capable of being glued to said flexible socket (4).

10. An orthopaedic appliance comprising a socket for receiving the distal end (7) of an amputation stump (6) and a device for heating and/or controlling the temperature of said socket according to anyone of the claims 1 to 9.

11. The orthopaedic appliance according to claim 10, **characterised in that** it is of one of the following types: lower limb prosthesis for fitting a tibial amputation stump, lower limb prosthesis for fitting a femoral amputation stump, upper limb prosthesis.
